# EUROPÄISCHE PATENTANMELDUNG

(11) **EP 2 174 618 A1**
(43) Veröffentlichungstag der Anmeldung: **14.04.2010**
(21) Anmeldenummer: 09170267.0
(22) Anmeldetag: 15.09.2009
(51) Int. Cl.: A61F 2/00

(54) **Katheter mit einer Applikationsvorrichtung für flüssige Wirkstoffe**

(30) Priorität: 13.10.2008 DE 102008042798
(71) Anmelder: Biotronik VI Patent AG, 6341 Baar (CH)
(72) Erfinder: Tittelbach, Michael, 90429, Nürnberg (DE)
(74) Vertreter: Lindner-Vogt, Karin L.

(57) **Zusammenfassung**

Die Erfindung betrifft einen Katheter (2), insbesondere einen endovaskulären Katheter, mit einer Applikationsvorrichtung (1) für flüssige Wirkstoffe (9) zu deren Aufbringen auf eine am Katheter angebrachte Wirkstoffspeicherzone.

## Beschreibung

Die Erfindung betrifft einen Katheter, insbesondere einen endovaskulären Katheter, mit einer Applikationsvorrichtung für flüssige Wirkstoffe zu deren Aufbringen auf eine am Katheter angebrachte Wirkstoffspeicherzone mit den im Oberbegriff des Anspruchs 1 angegebenen Merkmalen.

Zum Hintergrund der Erfindung ist festzuhalten, dass aus dem Stand der Technik Katheter mit sogenannten Drug-Eluting-Stents bekannt sind, bei denen ausschließlich Feststoffe als aktive pharmazeutische Wirkstoffe eingesetzt werden. Typische Lösungen für die Festlegung eines Wirkstoffes auf oder in Stents liegen in der Inkorporation des Wirkstoffes in einen polymeren Träger, das Einbringen in eine poröse Oberfläche oder das Integrieren in Wirkstoffdepots am Stent. Diese technischen Ausgestaltungen sind auf Feststoffe zugeschnitten, da flüssige Stoffe aus mechanischen Gründen nicht dauerhaft an der Stent-Oberfläche angebracht werden können. Ferner bringen Verbindungen eines flüssigen Stoffes mit dem Stent grundsätzliche Schwierigkeiten bezüglich der Lagerstabilität mit sich. So können Trocknungsphänomene oder Entmischungseffekte auftreten.

Ansätze zur Beschichtung von medizinischen Implantaten und insbesondere Stents mit Flüssigkeiten sind aus der US 2006/0124056 A1 oder der EP 1 325 758 A2 bekannt. Aus erstgenannter Druckschrift ist eine Vorrichtung zur Applizierung aktiver Substanzen auf Oberflächen von medizinischen Implantaten und insbesondere Stents bekannt, die aus einer Basisstation und einer austauschbaren Kartusche besteht. Auf der Kartusche ist ein Halter für die Stents vorgesehen, auf die über eine Düse die aktiven Substanzen auf die Oberfläche des Stents gesprüht werden. An der Basisstation ist eine Antriebseinheit vorgesehen, mittels der der Halter für die Stents und die Düse relativ zueinander verschoben werden, so dass eine definierte Benetzung der Stents mit Wirkstoffflüssigkeit zu gewährleisten versucht wird.

Bei der zweitgenannten Druckschrift wird die Dosiergenauigkeit des Flüssigmedikaments auf dem Stent durch Anlegen einer Potentialdifferenz zwischen der Sprüheinrichtung und dem Stent zu verbessern versucht.

Problematisch bei den vorstehenden Lösungen ist die Tatsache, dass ein operierender Arzt unmittelbar vor dem Setzen des Katheters mit Stent letzteren mit der Wirkstofflösung beschichten muss. Der Stent weist dazu eine poröse Oberfläche auf, bei der eine Lösung aus Alkohohl und beispielsweise Rapamycin aufgesprüht wird. Dies stellt gerade im OP-Umfeld eine komplizierte und zeitaufwendige Prozedur dar, bei der die Dosierung des Flüssigwirkstoffes sehr ungenau ist.

Davon ausgehend liegt der Erfindung die Aufgabe zugrunde, einen Katheter mit einer Applikationsvorrichtung für flüssige Wirkstoffe so auszugestalten, dass letztere in einfachen Prozessschritten und in kurzer Zeit mit einer definierten Wirkstoffdosis unmittelbar vor dem operativen Einsatz auf der Wirkstoffspeicherzone aufgebracht werden können.

Diese Aufgabe wird durch die Merkmale des Anspruchs 1 gelöst. Demnach weist die den Katheterkörper im Bereich der Wirkstoffspeicherzone mit Protektorhülle umgebende Applikationsvorrichtung einen zur Protektorhülle hin offenen oder zu öffnenden Wirkstoffbehälter vorzugsweise mit flexiblen, gegenüber der Protektorhülle und nach dem Abzug gegenüber dem Katheterkörper abdichtenden Begrenzungswänden beiderseits der Wirkstoffspeicherzone auf. Damit kann die Wirkstoffspeicherzone nach dem Abziehen der Protektorhülle mit dem im Wirkstoffbehälter befindlichen Wirkstoff beaufschlagt werden. Nach ausreichend langer Exposition der Wirkstoffspeicherzone in der Wirkstoffflüssigkeit kann der Wirkstoffbehälter vom Katheter abgezogen werden. Die im Wirkstoffbehälter enthaltene Flüssigkeit wird weitgehend darin zurückgehalten, so dass sie anschließend mit der Applikationsvorrichtung entsorgt werden kann. Die Wirkstoffspeicherzone am Katheter selbst ist durch eine Beaufschlagung über eine definierte Zeit mit einer genau festlegbaren Dosis des Wirkstoffes zu versehen. Der gesamte Applikationsvorgang kann dabei sehr einfach und in vergleichsweise kurzer Zeit durchgeführt werden, so dass der Katheter mit erfindungsgemäßer Applikationsvorrichtung eine besondere Eignung für das Aufbringen des Wirkstoffes unmittelbar vor dem operativen Einsatz des Katheters mit sich bringt. Unter "Wirkstoff" sind im Übrigen alle pharmazeutisch wirksamen Formulierungen zu verstehen, also z.B. Wirkstoffe in reiner Form, Lösungen von Wirkstoffen usw.

Bei der so bezeichneten "Wirkstoffspeicherzone" wird es sich in der Regel um die in der Beschreibungseinleitung bereits erwähnten wirkstoffbeladbaren Stents handeln. Darüber hinaus sind auch Katheter zum Weiten einer Stenose bekannt, bei denen lediglich das distale Ende des Katheters mit einem dort angeordneten Ballon an die verengte Stelle geführt und diese Stenose durch Dilatieren des Ballons aufgeweitet wird. Im Zusammenhang mit der vorliegenden Erfindung kann der Ballon selbst - also auch ohne darauf sitzendem Stent - als Wirkstoffspeicherzone fungieren.

Der unmittelbar vor dem operativen Einsatz aufgebrachte Wirkstoff wird dabei im Sinne einer "Speicherung" auf dem Stent oder Ballon gehalten, bis die Positionierung des Stents bzw. des Ballons des Katheters vonstatten gegangen ist.

In den abhängigen Ansprüchen sind bevorzugte Weiterbildungen des Katheters angegeben. Deren Merkmale, Einzelheiten und Vorteile sind der nachfolgenden Beschreibung entnehmbar, in der Ausführungsbeispiele des Erfindungsgegenstandes anhand der beigefügten Zeichnungen näher erläutert werden. Es zeigen:
- Fig. 1: einen schematischen, ausschnittsweisen Axialschnitt durch das distale Ende eines Katheters mit Applikationsvorrichtung in einem Lagerzustand,
- Fig. 2: einen vergrößerten Detailausschnitt gemäß der Einzelheit II gemäß Fig. 2,
- Fig. 3: eine Darstellung analog Fig. 1 mit abgezogener Protektorhülle in einem Ladezustand,
- Fig. 4: einen schematischen Axialschnitt der Applikationsvorrichtung in vom Katheter abgezogenen Entsorgungszustand,
- Fig. 5: eine Darstellung analog Fig. 1 eines Katheters mit einer Applikationsvorrichtung in einer alternativen Ausführungsform im Lagerzustand,
- Fig. 6: eine Darstellung analog Fig. 5 nach Abzug der Protektorhülle in einem Zwischenzustand, und
- Fig. 7: eine Darstellung analog Fig. 5 mit aus dem Vorratsbehälter in den Wirkstoffbehälter übergeführter Wirkstoffflüssigkeit im Ladezustand.

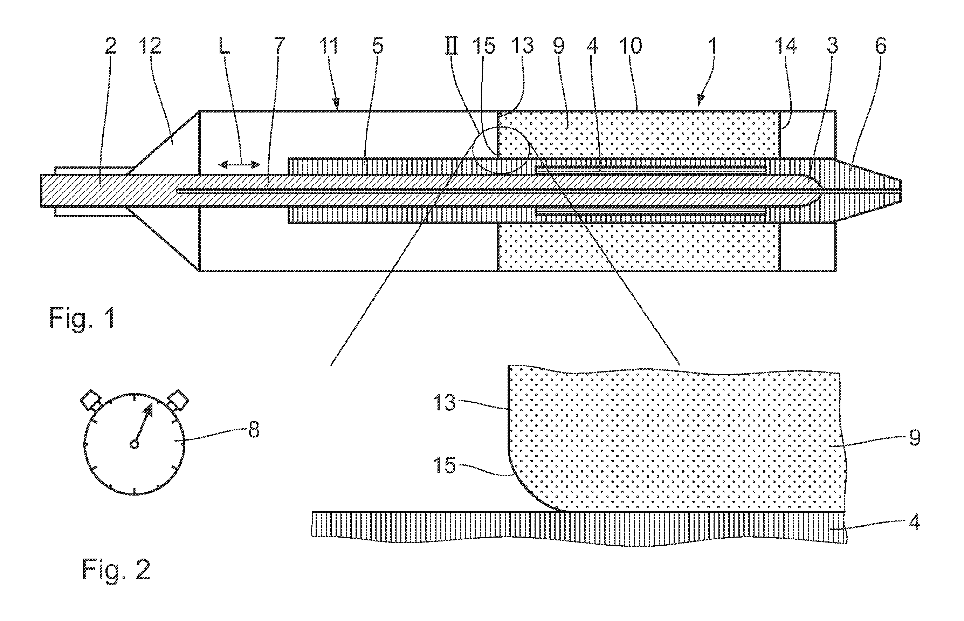

Anhand von Fig. 1 und 2 ist ein Katheter mit einer als Ganzes mit 1 bezeichneten Applikationsvorrichtung in einer ersten Ausführungsform im Lagerzustand zu beschreiben. So weist der Katheter einen langgestreckten Katheterkörper 2 auf, von dem nur der Bereich vor dem distalen, in den Körper eines Patienten einzuführenden Ende 3 dargestellt ist. In üblicher Weise ist vor diesem distalen Ende 3 ein Stent 4 auf den nicht dargestellten Ballon des Katheterkörpers 2 aufgecrimpt und von einer Protektorhülle 5 abgedeckt. Letztere besteht aus einem schlauchförmigen Hauptabschnitt, an der distal ein Schrumpfschlauchstück 6 zur Anbringung eines Drahtes 7 der Protektorhülle 5 befestigt ist. Letzterer ist zur ausreichend stabilen Halterung der Protektorhülle 5 in ein nicht näher dargestelltes Drahtlumen des Katheterkörpers 2 eingeschoben. Die Protektorhülle 5 steht beiderseits in Längsrichtung L des Katheterkörpers 2 über den Stent 4 hinaus.

Über der Protektorhülle 5 ist die Applikationsvorrichtung 1 für einen flüssigen Wirkstoff 9 platziert. Kernbestandteil dieser Applikationsvorrichtung 1 ist ein zur Protektorhülle 5 hin offener Wirkstoffbehälter 10, der als ringförmiges Gefäß um die Protektorhülle 5 herum angeordnet ist. Gehalten ist der Wirkstoffbehälter 10 an einer hülsenartigen, längeren Halterung 11, die mit einem Fixierkonus 12 an ihrem proximalen Ende auf dem Katheterkörper 2 sitzt. Am distalen Ende ist die Halterung 11 auf der Protektorhülle 5 in geeigneter Weise lösbar befestigt.

Der Wirkstoffbehälter 10 weist proximal und distal jeweils eine ringförmig umlaufende, flexible Begrenzungswand 13, 14 auf, die mit ihrer radial nach innen weisenden Stirnkante als Dichtlippe 15 jeweils gegen Flüssigkeitsdurchtritt abdichtend am Umfang der Protektorhülle 5 anliegt. Wie aus der vergrößerten Detaildarstellung gemäß Fig. 2 erkennbar ist, kann zur Verbesserung des Dichtkontaktes die Dichtlippe 15 der Begrenzungswände 13, 14 flexibel umgebogen sein.

In dem in Fig. 1 gezeigten Lagerzustand des Katheters mit Applikationsvorrichtung 1 ist der Stent durch die Protektorhülle 5 vom Wirkstoff 9 im Wirkstoffbehälter 10 getrennt. Die gesamte Vorrichtung mit Flüssigwirkstoff 9 kann nach Herstellung beispielsweise durch Strahlensterilisation sterilisiert werden. Dazu ist es notwendig, dass insbesondere der Wirkstoff 9 durch eine Strahlensterilisation nicht in seiner Wirkung beeinträchtigt wird.

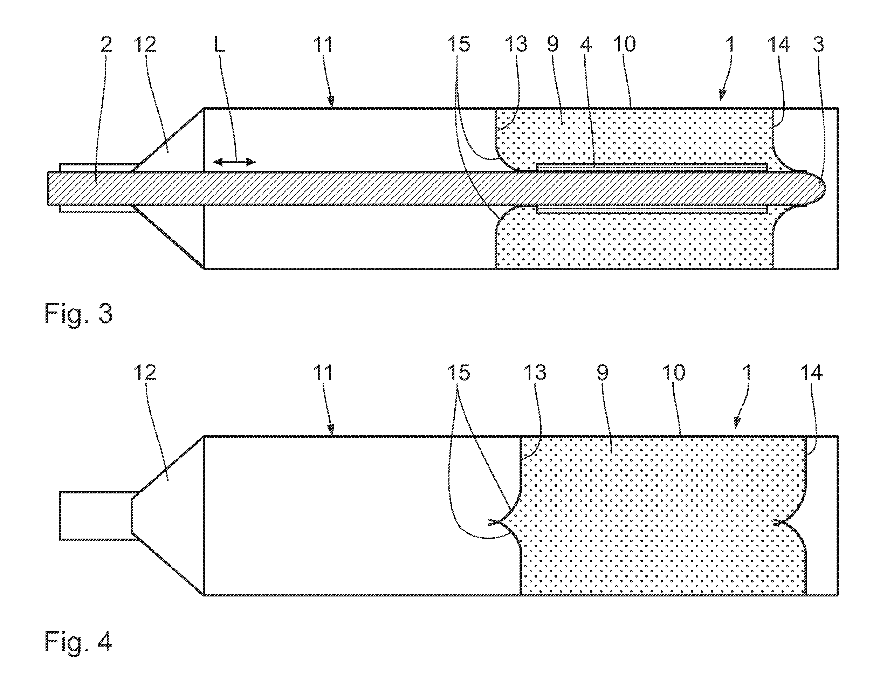

Zur Aufdosierung des Wirkstoffes 9 auf den Stent 4 wird während einer Operation die Protektorhülle 5 in distaler Richtung vom Katheterkörper 2 und der Applikationsvorrichtung 1 abgezogen, wobei die beispielsweise nach Art eines Septums ausgebildeten Begrenzungswände 13, 14 unter Dichthalten an der Oberfläche der Protektorhülle 5 entlang gleiten. Nach Entfernen der Protektorhülle 5 dichten die Begrenzungswände 13, 14 gegen den Katheterkörper 2 beiderseits des Stents 4 ab, so dass in diesem Ladezustand - wie in Fig. 3 gezeigt ist - der Wirkstoff 9 nun auf den Stent 4 einwirken und auf diesem aufdosiert werden kann. Mit Hilfe einer den Katheter mit Applikationsvorrichtung 1 zugeordneten Stoppuhr 8, wie sie beispielsweise in ein Operationsset integriert werden kann, kann eine definierte Beaufschlagungszeit des Stents 4 mit dem flüssigen Wirkstoff 9 bestimmt werden. Die Stoppuhr 8 kann dabei durch das Entfernen der Protektorhülle 5 automatisch gestartet werden. Nach Ablauf der definierten Beaufschlagungszeit kann ein Alarm generiert werden. Der Operateur wird die gesamte Halterung 11 mit dem Wirkstoffbehälter 10 dann vom distalen Ende 3 des Katheterkörpers 2 abziehen, wobei die proximale Begrenzungswand 13 über den Stent 4 streift und somit überschüssige Wirkstoffflüssigkeit mitnimmt.

Wie aus Fig. 4 deutlich wird, sind die Begrenzungswände 13, 14 so ausgelegt, dass sie auch nach dem Abzug der Applikationsvorrichtung 1 vom Katheter für eine Abdichtung des Wirkstoffbehälters 10 nach außen hin sorgen, so dass der flüssige Wirkstoff 9 zusammen mit der Applikationsvorrichtung 1 sauber entsorgbar ist.

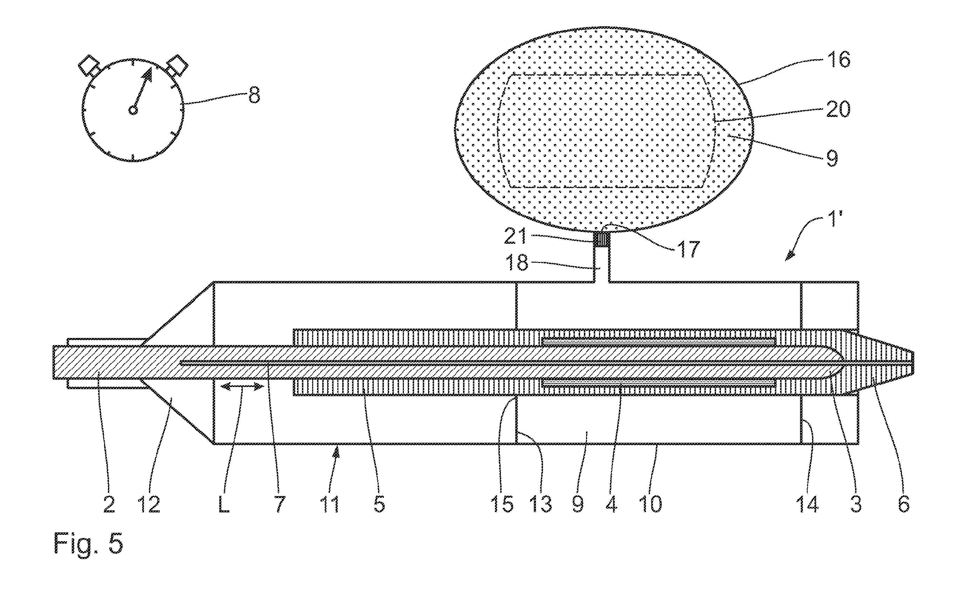

In den Fig. 5 bis 7 ist eine alternative Ausführungsform eines Katheters mit Applikationsvorrichtung 1' gezeigt. Der eigentliche Katheter mit Katheterkörper 2, Stent 4 und Protektorhülle 5 einerseits sowie die Grundkonfiguration der Applikationsvorrichtung 1' mit Wirkstoffbehälter 10 und Halterung 11 ist gegenüber dem Ausführungsbeispiel gemäß den Fig. 1 bis 4 unverändert. Insoweit sind übereinstimmende Komponenten mit identischen Bezugszeichen versehen und es kann auf die dortige Beschreibung verwiesen werden.

Im Unterschied zum vorherigen Ausführungsbeispiel ist bei der Applikationsvorrichtung 1' ein separater Vorratsbehälter 16 vorgesehen, der als flexibler Beutel ausgebildet und mit einer zerreißbaren Membran 17 verschlossen ist. Der Vorratsbehälter 16 steht über einen Schlauch 18 mit dem im Lagerzustand gemäß Fig. 5 noch leeren Wirkstoffbehälter 10 in Verbindung.

Aufgrund der vorstehenden Konfiguration ist es möglich, den Wirkstoff 9 unter sterilen Bedingungen herzustellen und anschließend in den sterilen Vorratsbehälter 16 einzufüllen. Eine nachträgliche Sterilisation des Vorratsbehälters 16 mit dem Wirkstoff 9 ist dann nicht mehr notwendig, so dass auf eine möglicherweise den Wirkstoff beeinträchtigende Strahlsterilisation verzichtet werden kann. Das gesamte System kann abschließend beispielsweise mittels Gassterilisation mit Ethylenoxid sterilisiert werden. Es genügt dazu, den Vorratsbehälter 16 gasdicht auszuführen.

Für das Aufdosieren des Wirkstoffes 9 auf den Stent 4 wird - wie beim Ausführungsbeispiel gemäß den Fig. 1 bis 4 - die Protektorhülle 5 aus der Applikationsvorrichtung 1' herausgezogen, die Begrenzungswände 13, 14 schließen mit ihren Dichtlippen 15 dann nach wie vor dicht zum Katheterkörper 2 hin ab.

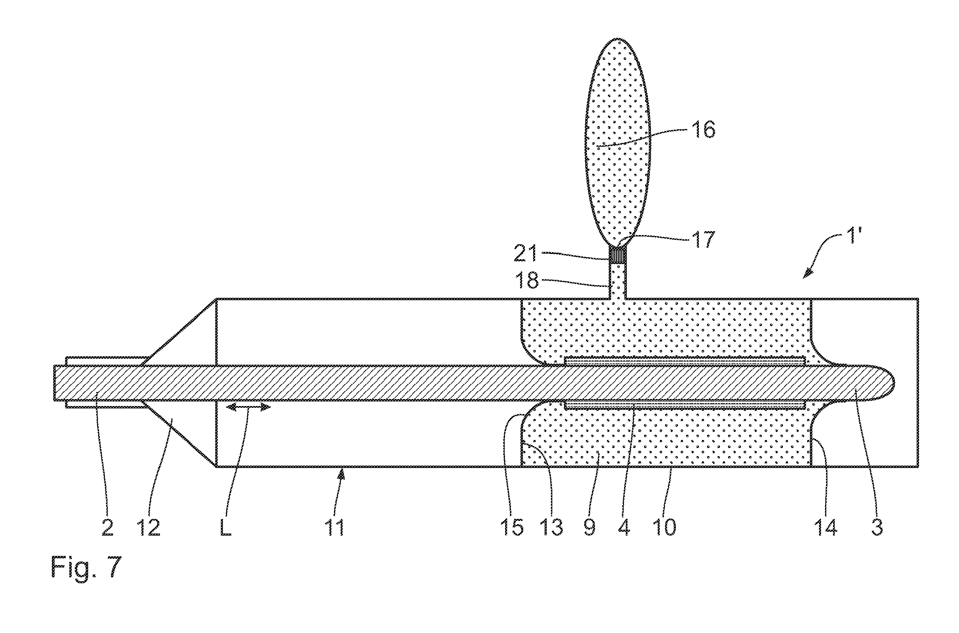

Anschließend wird der beutelartige Vorratsbehälter 16 von Hand unter Druck gesetzt, die Membran 17 zerreißt und der Wirkstoff 9 gelangt über den Schlauch 18 in den Wirkstoffbehälter 10, womit der Stent 4 wiederum über eine definierte Zeit mit dem Wirkstoff beaufschlagbar ist. Die Beaufschlagungszeit kann wieder mit der in Fig. 7 schematisch angedeuteten Stoppuhr 8 gemessen werden.

Nach Ablauf der Beaufschlagungszeit wird analog dem Ausführungsbeispiel gemäß Fig. 1 bis 4 die ganze Applikationsvorrichtung 1' zusammen mit dem Vorratsbehälter 16 vom distalen Ende 3 des Katheterkörpers 2 abgezogen, wobei wiederum der Wirkstoff 9 mitgenommen und vom Stent 4 abgestreift wird. Der Katheter kann anschließend direkt gesetzt und der wirkstoffbeladene Stent 4 appliziert werden.

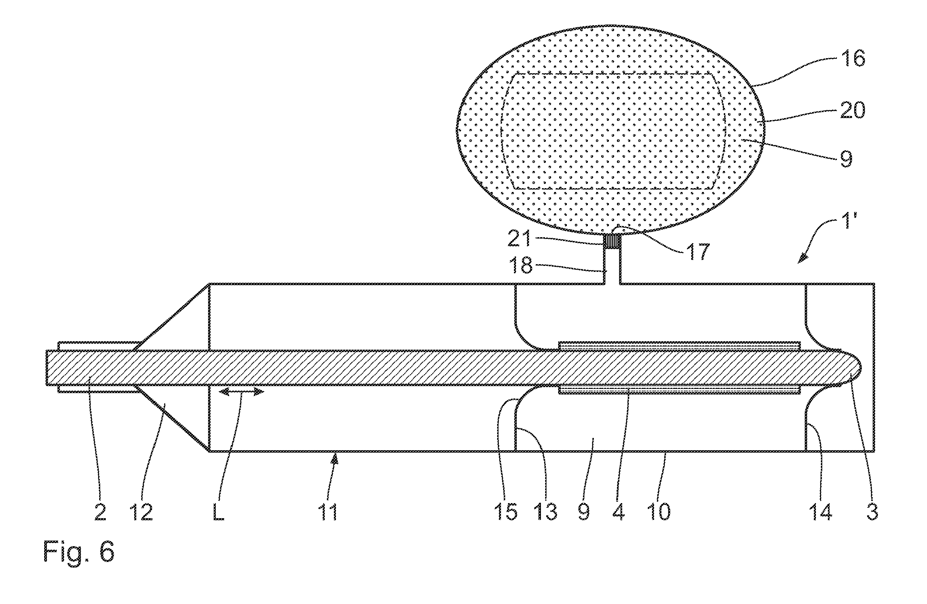

Wie in Fig. 5 und 6 gestrichelt angedeutet ist, kann innerhalb des beutelartigen Vorratsbehälters 16 noch ein starrer Innenbehälter 20 beispielsweise in Form einer Glasampulle für die Lagerung des Wirkstoffs 9 vorgesehen sein, falls letzterer optimal gegen Degradation z.B. aufgrund eines Sterilisationsprozesses mit Ethylenoxid zu schützen ist. Die Glasampulle kann durch den Beutel hindurch aufgebrochen werden, wodurch sich der Wirkstoff 9 in den Beutel und weiter in den Wirkstoffbehälter 10 ergießt.

Um durch das Aufbrechen der Glasampulle möglicherweise entstehende Glas und andere Partikel im Vorratsbehälter 16 zurückzuhalten, ist im Schlauch 18 ein Filter 21 vorgesehen.

Es ist zu erwähnen, dass der Wirkstoffbehälter auch als um die Protektorhülle ringförmig herum gelegter Schlauchbeutel ausgeführt werden kann, dessen an die Protektorhülle angrenzende Wand bei dessen Abziehen mit herausgerissen wird, um den Zugang des Wirkstoffes zum Stent zu ermöglichen. Schließlich ist fest zu halten, dass die erfindungsgemäße Applikationsvorrichtung bei jedweder Art von auf einem Katheter sitzenden medizinischen Gerät, ob dauerhaft oder temporär in den Körper einbringbar, wie beispielsweise unter Weglassung des eigentlichen Stents ein Ballon zur Aufweitung einer Stenose, einsetzbar und das Gerät so mit einem Wirkstoff zu beladen ist. Dies wird durch die so bezeichnete Wirkstoffspeicherzone umschrieben.

## Patentansprüche

1. Katheter, insbesondere endovaskulärer Katheter, mit einer Applikationsvorrichtung für flüssige Wirkstoffe auf eine am Katheter vorgesehene Wirkstoffspeicherzone, umfassend
- einen langgestreckten Katheterkörper (2) mit einem distalen Ende (3),
- die vor dem distalen Ende (3) am Katheterkörper (2) befindliche Wirkstoffspeicherzone (4), und
- eine die Wirkstoffspeicherzone (4) umgebende, abziehbare Protektorhülle (5), **gekennzeichnet durch**
- die den Katheterkörper (2) im Bereich der Wirkstoffspeicherzone (4) mit Protektorhülle (5) umgebende Applikationsvorrichtung (1, 1'), die einen zur Protektorhülle (5) hin derart offenen oder zu öffnenden Wirkstoffbehälter (10) aufweist, dass die Wirkstoffspeicherzone (4) nach dem Abziehen der Protektorhülle (5) mit dem im Wirkstoffbehälter (10) befindlichen Wirkstoff (9) unmittelbar vor einem Einsatz des Katheters beaufschlagbar ist.

2. Katheter nach Anspruch 1, **dadurch gekennzeichnet, dass** die Applikationsvorrichtung (1, 1') mit flexiblen, gegenüber der Protektorhülle (5) und nach deren Abzug gegenüber dem Katheterkörper (2) abdichtenden Begrenzungswänden (13, 14) beiderseits der Wirkstoffspeicherzone (4) versehen ist.

3. Katheter nach Anspruch 1 oder 2, **dadurch gekennzeichnet, dass** die Applikationsvorrichtung (1, 1') eine hülsenartige Halterung (11) aufweist, die
- den Wirkstoffbehälter (10) aufnimmt,
- mit einem proximalen Ende am Katheterkörper (2) befestigt und
- mit einem distalen Ende auf der Protektorhülle (5) unter Freilassung deren distalen Endes sitzt.

4. Katheter nach Anspruch 2 oder 3, **dadurch gekennzeichnet, dass** die Begrenzungswände (13, 14) durch ein Septum gebildet sind und/oder dass die Begrenzungswände (13, 14) eine Dichtlippe (15) zur Protektorhülle hin aufweisen.

5. Katheter nach einem der vorgenannten Ansprüche, **dadurch gekennzeichnet, dass** die Begrenzungswände (13, 14) derart ausgelegt sind, dass auch nach dem Abzug der Applikationsvorrichtung (1, 1') vom Katheterkörper (2) der Wirkstoffbehälter (10) nach außen dicht bleibt.

6. Katheter nach einem der vorgenannten Ansprüche, **dadurch gekennzeichnet, dass** der Wirkstoffbehälter ein die Protektorhülle (5) mit Wirkstoffspeicherzone (4) umgebender Ringschlauch ist, dessen zur Protektorhülle (5) hin gewandte Innenwand mit dem Abzug der Protektorhülle (5) entfernbar ist.

7. Katheter nach einem der vorgenannten Ansprüche, **dadurch gekennzeichnet, dass** er komplett mit Wirkstoff-gefüllter Applikationsvorrichtung (1) sterilisierbar ist.

8. Katheter nach einem der Ansprüche 1 bis 6, **gekennzeichnet durch** einen separaten, mit Wirkstoff (9) gefüllten Vorratsbehälter (16), der vor Applikation mit dem Wirkstoffbehälter (10) der Applikationsvorrichtung (1') zum Einfüllen des Wirkstoffes (9) in den Wirkstoffbehälter (10) verbindbar ist.

9. Katheter nach Anspruch 8, **dadurch gekennzeichnet, dass** zur Verbindung zwischen Vorratsbehälter (16) und Wirkstoffbehälter (10) ein Schlauch (18) vorgesehen ist, wobei im Schlauch (18) ein Partikel-Filter (21) anbringbar ist.

10. Katheter nach Anspruch 8 oder 9, **dadurch gekennzeichnet, dass** der Vorratsbehälter (16) mit einer zerreißbaren Membran (17) verschlossen ist.

11. Katheter nach einem der Ansprüche 8 bis 10, **dadurch gekennzeichnet, dass** der Vorratsbehälter (16) als Beutel ausgebildet ist.

12. Katheter nach Anspruch 11, **dadurch gekennzeichnet, dass** im beutelartigen Vorratsbehälter (16) ein starrer, aufbrechbarer Innenbehälter (20) vorgesehen ist.

13. Katheter nach einem der Ansprüche 8 bis 12, **dadurch gekennzeichnet, dass** der separate Vorratsbehälter (16)
- mit Wirkstoff (9) getrennt vom Katheter mit Applikationsvorrichtung (1') sterilisiert ist oder
- unter sterilen Bedingungen hergestellten und in den Vorratsbehälter (16) abgefüllten Wirkstoff enthält.

14. Katheter nach einem der vorgenannten Ansprüche, **dadurch gekennzeichnet, dass** die Wirkstoffspeicherzone durch einen Stent (4) oder einen dilatierbaren Ballon am Katheter gebildet ist.

15. Katheter nach einem der vorgenannten Ansprüche, **dadurch gekennzeichnet, dass** der Applikationsvorrichtung (1, 1') eine Stoppuhr (8) zur Messung der Beaufschlagungszeit des Stents (4) mit dem Wirkstoff (9) zugeordnet ist.
